# EUROPEAN PATENT APPLICATION

(11) **EP 2 679 231 A1**
(43) Date of publication of application: **01.01.2014**
(21) Application number: 11859272.4
(22) Date of filing: 25.02.2011
(51) Int. Cl.: A61K 35/50, A61K 35/12, A61K 9/08, A61P 13/10, A61P 13/02, A61P 13/00

(54) **AGENT FOR TREATING URINARY INCONTINENCE INCLUDING STEM CELLS DERIVED FROM AMNIOTIC FLUID**

(71) Applicant: Kyungpook National University Hospital, Jung-gu, Daegu 700-721 (KR)
(72) Inventor: LIM, Jeong Ok, Daegu 706-948 (KR); KWON, Tae Gyun, Daegu 706-102 (KR); CHUN, So Young, Daegu 706-010 (KR); YOO, James J., Winston Salem, NC 27104 (US)
(74) Representative: Nordic Patent Service A/S
(86) International application number: PCT/KR2011/001368
(87) International publication number: WO 2012/115298

(57) **Abstract**

The present invention relates to a cell therapy product which is intended for regenerating a sphincter muscle and which contains stem cells derived from amniotic fluid, and more particularly, to a cell therapy product which is intended for regenerating the sphincter vesicae and which contains stem cells derived from amniotic fluid. Also, the cell therapy product of the present invention can be provided in the form of a formulation for administration through injection, said formulation being injected into a hydrogel complex to thereby improve the effects thereof. The composition including stem cells derived from amniotic fluid according to the present invention enables stem cells to be differentiated into muscles in the body of individual suffering from urinary incontinence by directly injecting the composition into the individual, thus effectively controlling urinary incontinence by recovering muscle functions. That is, the stem cells derived from amniotic fluid of the present invention are differentiated into muscles in-situ, and the differentiation into muscles can thus be achieved only with cells in order to recover muscle functions.

## Description

### [Technical Field]

The present invention relates to a cellular therapeutic agent containing amniotic fluid-derived stem cells for sphincter regeneration and, more particularly, to a cellular therapeutic agent containing amniotic fluid-derived stem cells for urethral sphincter regeneration. Moreover, the present invention relates to a cellular therapeutic agent containing amniotic fluid-derived stem cells for treating urinary incontinence.

The cellular therapeutic agent of the present invention is formulated into a dosage form for injection. Moreover, the amniotic fluid-derived stem cells of the present invention may preferably be mixed with a hydrogel complex.

Furthermore, the present invention relates to an optimum medium that induces differentiation of amniotic fluid stem cells into myocytes and, more particularly, to a medium containing 5-azaC or TGF-β, preferably, skeletal muscle supernatant, in skeletal muscle differentiation medium.

### [Background Art]

Stem cells refer to cells having the ability of self-replication and the ability of differentiation into at least two cells and can be divided into totipotent stem cells, pluripotent stem cells, and multipotent stem cells.

Totipotent stem cells are cells with totipotent properties capable of developing into one perfect individual, and these properties are possessed by cells up to the 8-cell stage after the fertilization of an oocyte and a sperm. When these cells are isolated and transplanted into the uterus, they can develop into one perfect individual. Pluripotent stem cells, which are cells capable of developing into various cells and tissues derived from ectodermal, mesodermal and endodermal layers, are derived from an inner cell mass located inside blastocysts generated 4-5 days after fertilization. These cells are called embryonic stem cells and can differentiate into various other tissue cells but not form new living organisms. Multipotent stem cells are stem cells capable of differentiating into only cells specific to tissues and organs containing these cells.

The multipotent stem cells were first isolated from adult bone marrow (Y. Jiang et al., Nature, 418:41, 2002), and then also found in other various adult tissues (C. M. Verfaillie, Trends Cell Biol., 12:502, 2002). In other words, although bone marrow is the most widely known source of stem cells, the multipotent stem cells were also found in the skin, blood vessels, muscles and brains (J. G. Tomas et al., Nat. Cell Biol., 3:778, 2001; M. Sampaolesi et al., Science, 301:487, 2003; Y. Jiang et al., Exp. Hematol., 30:896, 2002). However, stem cells are very rarely present in adult tissues, such as bone marrow, and such cells are difficult to culture without inducing differentiation, and thus difficult to culture in the absence of specifically screened media. That is, it is very difficult to maintain the isolated stem cells *in vitro.*

Stem cells derived from human amniotic fluid surrounding the fetus can be easily obtained during pregnancy and childbirth, can proliferate in large quantities, and do not cause any tumor formation after transplantation into animals like embryonic stem cells, which are the most important advantages. In particular, human amniotic fluid stem cells have no ethical issues that embryonic stem cells possess and thus are the most potential stem cells that can be used in cell therapy. Cell sources such as autologous muscular tissue, bone marrow, fat, bone, etc. are used, but most are collected by invasive methods which carry significant complications such as bleeding, infection, damage to organs, etc, and thus a stem cell source that can be obtained by non-invasive methods is required. Amniotic fluid stem cells can be easily obtained from the routine inspection during pregnancy and during childbirth, can proliferate in large quantities, and do not require immunosuppressive agents as they are non-immunogenic. Compared to embryonic stem cells, amniotic fluid stem cells do not cause any tumor formation after transplantation into animals, have no ethical issues, and can differentiate into all organs due to their pluripotential capability, thus serving as an ideal stem cell source for the treatment of diseases.

Meanwhile, urinary incontinence in women is caused by sagging of the urethra and bladder, which results from weakening of pelvic floor muscles arising from pudendal nerve injury due to frequent childbirth and aging. Currently, the number of female urinary incontinence patients in Korea is estimated to be about 4 to 5 million people and is increasing every year due to a rapid increase in the number of old age women. Thus, female urinary incontinence is one of the serious social problems all over the world. To treat urinary incontinence patients, surgical therapy or injection therapy for supporting the urethra and bladder are used. Currently, the surgical therapy which is an invasive method has a problem in that complications can occur, and the injection therapy has problems in that it employs expensive substances, and thus cannot be easily applied to all patients, and in that it has a success rate of only 50-60%, such that injection and surgery are required again.

Stem cell injection therapy does not need anesthesia and enables easy injection of stem cells into urethral sphincter. Thus, if the stem cell injection therapy can improve the contractility of urethral sphincter and increase leak point pressure, the stem cell therapy can be advantageously used to treat urinary incontinence.

In recent years, cellular therapies for urethral sphincter regeneration using these stem cells have been actively applied in the treatment of urinary incontinence. Korean Patent Publication No. 10-2009-0056925 discloses a cellular therapeutic agent for treating urinary incontinence using fat-derived stem cells, and Korean Patent Publication No. 10-2010-0018655 discloses a method for treating sphincter deficiency using differentiated immature adipocytes, but they do not yet provide satisfactory therapeutic effects.

Accordingly, the present inventors have made great efforts to develop an agent for treating urinary incontinence and found that amniotic fluid-derived stem cells are effective for the treatment of urinary incontinence, thereby completing the present invention.

### [Disclosure]

### [Technical Problem]

Accordingly, the present invention has been made to solve the above-described problems, and an object of the present invention is to a composition containing amniotic fluid-derived stem cells for treating sphincter deficiency, and a therapeutic method.

Moreover, another object of the present invention is to provide an injectable formulation containing amniotic fluid-derived stem cells, which is directly inject into a sphincter deficiency area to differentiate in situ into myocytes, thus stimulating urethral sphincter regeneration.

### [Technical Solution]

To accomplish the above objects, the present invention provides a composition containing amniotic fluid-derived stem cells for treating sphincter deficiency. The composition of the present invention can be used for the treatment of urinary incontinence.

Moreover, the amniotic fluid-derived stem cells of the present invention may be mixed with a hydrogel complex and injected into an affected area, thus increasing in situ differentiation in the affected area. Cell injectable hydrogel of the present invention contains the stem cells to simply inject the cells into the body without any surgical operation and to help the cells stay in one place of the body, thus facilitating the differentiation or organization of the cells in the body. Preferably, the hydrogel of the present invention contains an alginate/PF-127/hyaluronic acid complex. Moreover, the mixing ratio of the respective polymers in the complex may preferably be 6:6:1.

Furthermore, the amount of stem cells injected according to the present invention may preferably be 10⁵ to 10⁶ cell/sphincter, more preferably, 10⁶ cell/sphincter.

As used herein, the term "stem cell" refers to a master cell that can reproduce indefinitely to form the specialized cells of tissues and organs. The stem cells are developmentally pluripotent and multipotent stem cells. A stem cell can divide to produce two daughter stem cells, or one daughter stem cell and one progenitor ("transit") cell, which then proliferates into the tissue's mature, fully formed cells.

As used herein, the term "differentiation" refers to a phenomenon in which the structure or function of cells is specialized during the division, proliferation and growth thereof, that is, the feature or function of cell or tissue of an organism changes in order to perform work given to the cell or tissue. Generally, it refers to a phenomenon in which a relatively simple system is divided into two or more qualitatively different partial systems. For example, it means that a qualitative difference between the parts of any biological system, which have been identical to each other at the first, occurs, for example, a distinction such as a head or a body between egg parts, which have been qualitatively identical to each other at the first in ontogenic development, occurs, or a distinction such as a muscle cell or a nerve cell between cells occurs, or the biological system is divided into qualitatively distinguishable parts or partial systems as a result thereof.

As used herein, the term "cellular therapeutic agent" refers to a drug used for the purpose of treatment, diagnosis and prevention, which contains a cell or tissue prepared through isolation from humans, culture and specific operation (as provided by the US FDA). Specifically, it refers to a drug used for the purpose of treatment, diagnosis and prevention through a series of behaviors of *in vitro* multiplying and sorting living autologous, allogenic and xenogenic cells or changing the biological characteristics of cells by other means for the purpose of restoring the functions of cells and tissues. Cellular therapeutic agents are broadly divided, according to the differentiation level of cells, into somatic cell therapeutic agents and stem cell therapeutic agents. The present invention particularly relates to a cellular therapeutic agent containing amniotic fluid-derived stem cells.

The inventors of the present invention have conducted experiments by directly injecting amniotic fluid-derived stem cells into urinary incontinence animal models to determine whether the stem cells would differentiate into muscles in the body to restore normal muscle function for controlling urinary incontinence and completed the present invention. The injection application of stem cells and the evaluation of in situ differentiation performed in the present invention have clinical significance. This is because the injected stem cells underwent proliferation *in vitro* only without gene manipulation or differentiation and were simply injected by injection without any invasive process such as surgery and it was proved that the injected stem cells differentiated in situ in urethral sphincter, a target tissue.

Moreover, in the present invention, various cell culture media were used for the differentiation of amniotic fluid stem cells into muscles, from which it was found that the conditioned medium (obtained from human skeletal muscle culture medium) was the best among others, and better functional restoration was observed when cells were added to an alginate/pluronic acid/hyaluronic acid hydrogel complex and injected into a damaged area, while the urethral function was restored even in the case of the medium containing cells only.

Furthermore, three types of differentiation media were used to induce the differentiation of amniotic fluid stem cells into muscles by *in vitro* experiment, and the differentiation capability was evaluated by morphological, genetic, and immunochemical methods. To find the optimal differentiation conditions, a medium in which 5-azaC or TGF-β was added to regular myogenic medium and a medium containing human skeletal muscle supernatant were used. The supernatant medium was evaluated as the most suitable medium for the differentiation of amniotic fluid stem cells from the results of the analysis based on the cell proliferation rate and the expression of genes and proteins associated with myogenic differentiation.

In the present invention, in an *in vivo* experiment using urinary incontinence mouse models, non-differentiated amniotic fluid stem cells were grafted onto a hydrogel and injected into urethral sphincter with weakened muscle function. A sham-operation group was established as a positive control, and a non-treated group and a cell group that was not treated with hydrogel were established as a negative control. Urodynamic factors such as urethral closing pressure and leak point pressure were measured from the animals in each group at one, two, and four weeks after operation, and the reconstruction of urethral sphincter was evaluated through histological analysis. Significant recovery of urethral closing pressure and leak point pressure was observed in the experimental group compared to the control group, and the engraftment of amniotic fluid stem cells and the differentiation into surrounding myocytes were observed in the histological test. Thus, it was found that the amniotic fluid stem cells can easily differentiate into myocytes, have high efficiency during differentiation with human skeletal muscle supernatant, and have significant in situ differentiation *in vivo* when grafted onto the hydrogel.

### [Advantageous Effects]

The composition containing amniotic fluid stem cells of the present invention is directly injected into a urinary incontinence individual such that the stem cells differentiate into muscles to effectively control urinary incontinence, thus restoring muscle function. The amniotic fluid stem cells of the present invention can differentiate in situ into muscles *in vivo* to restore muscle function. Moreover, when a biocompatible cellular injectable hydrogel is injected together with cells, the functional restoration can be further improved.

### [Description of Drawings]

FIG. 1 shows the characteristics of hAFSCs by FACS, in which FIG. 1A shows antigen markers expressed by stem cells derived from human amniotic fluid, and FIG. 1B shows that more than 90% Class II c-kit (+) cells are positive for c-kit.
FIG. 2 shows the myogenic differentiation of hAFSCs *in vitro*, in which FIG. 2A shows the morphological change in different myogenic conditions, FIG. 2B shows the cell viability through CCK8 assay, FIG. 2C shows the expression of stem cell and early myogenic markers, FIG. 2D shows the results of double staining of primary antibodies and nucleic acid staining DAPI, and FIG. 2E shows the control of differentiation into myoblasts.
FIG. 3A shows the visualization of the presence, migration, and duration of injected hAFSCs, FIG. 3B shows the effects of urethral sphincter regeneration medicated by injected cells, FIG. 3C shows the results of histological analysis of urethral sphincter regeneration, FIG. 3D shows the results of immunohistochemical analysis of Nestin, MyoD, α-SM actin and α-actinin, FIG. 3E shows the results of real-time PCR analysis, and FIG. 3F shows the results of myogenic gene expression analysis using mouse primers.
FIG. 4 shows the results of functional immunoassay of hAFSCs, in which FIG. 4A shows the analysis results of surface phenotypes and FIG. 4B shows the result of immunohistochemical staining of urethral sphincter sections on CD8 active lymphocytes.
FIG. 5 shows confocal laser scanning microscopic images of hAFSCs labeled with MNP_{S}@SiO₂ (RITC), in which FIG. 5A shows the images at various concentrations of MNPs@SiO₂ (RITC), FIG. 5B shows the ratio of labeled hAFSCs in the entire cells, FIG. 5C shows the results of cytotoxicity of MNPs@SiO₂ (RITC), and FIG. 5D shows optical images of nanoparticle-labeled hAFSCs.
FIG. 6 is a graph showing the increase in LPP and CP by two types of hAFSCs.
FIG. 7 is a graph showing the significantly increase in LPP and CP by hAFSCs mixed with a hydrogel containing alginate, pluronic acid and hyaluronic acid.

### [Mode for Invention]

Hereinafter, the present invention will be described in detail with reference to the following Examples. However, these Examples are illustrative of the present invention, and the scope of the present invention is not limited to these Examples.

### Example 1: Characteristics of Human Amniotic Fluid Cells and Isolation of AFSCs

The present study and the use of human amniotic fluid was approved by the Ethics Committee of the Medical School of Kyungpook National University.

Amniotic fluids were obtained from women undergoing routine amniocentesis at a gestational age of 15 to 19 weeks after informed consent. Amniotic fluids containing cells were cultured on cover glass in Amino MAXTMII (Gibco-Invitrogen, Grand Island, NY) for at least two week. Cells were harvested with trypsin/EDTA solution and cultured with Chang Medium (α-MEM, 15% ES-FBS, 1% glutamine, and 1% penicillin/streptomycin, Gibco), 18% Chang B, and 2% Chang C (Irvine Scientific, Irvine, CA) at 37 °C under 5% CO₂ in petri dishes. The cells were maintained at 70-80% confluence without any feeder layer.

The cultured amniotic fluid cells (passage 3) were analyzed by fluorescence activated cell sorter (FACS, BD Biosciences, San Jose, CA) using various surface antigens and cellular markers. Embryonic stem cells (c-kit, SSEA-4, Oct-3/4), mesenchymal stem cells (CD44, CD45, CD73, CD90, CD105), and immunogenic markers (HLA-ABC, -DR) were used for the characterization of the cells (all BD). Isotype immunoglobulin for each antibody was used as an internal control. Stem cell group was isolated with c-kit antibodies. C-kit (+) cells were cultured with Chang Medium in petri dishes and maintained up to 4 passages. These cells were sorted with c-kit and named human amniotic fluid stem cells (hAFSCs).

### Example 2: Differentiation of hAFSCs into myocytes in vivo

Three different types of myogenic media were used to differentiate hAFSCs into myocyte-like cells: (i) myogenic medium (0.5% chick embryo extract, 10% horse serum, 1% penicillin/streptomycin, DMEM low glucose, all from Gibco-Invitrogen) treated with 3 mM 5-aza-20-deoxycytidine (5-azaC; Sigma-Aldrich, St. Louis, MO); (ii) myogenic medium treated with TGF-β (5ng/ml, Peprotech, Rocky Hill, NJ); and (iii) conditioned medium (CM) (obtained from human skeletal muscle cell culture medium).

hAFSCs were seeded in culture medium at a density of 3,000 cells/cm² and cultured with Chang medium for 24 hours. Then, the medium was replaced with myogenic medium. After 24 hour culture, the myogenic medium was replaced with myocyte culture medium. Cells were grown up to 14 days for analysis. Under 5-azaC condition, Matrigel pre-coated dishes (BD Biosciences) were used.

Cell viability at 14 days was measured using a CCK-8 assay kit (Dojindo, Japan). The genotypic and morphologic conversion of hAFSCs into myogenic lineage cells was analyzed by real-time polymerase chain reaction (PCR) and immunocytochemical (ICC) staining. Total RNAs were extracted from the cultured cells using a TRI Reagent (Invitrogen). Primers were designed using Primer Express (Applied Biosystems, Warrington, UK). The sequences of stem cell and myogenic specific markers (Pax7, Myf-5, MyoD, Desmin, Dystrophin, Myogenin, α-actinin, and α-SM actin) are listed in Table 1.

**[Table 1]**

| Gene | Sequences |
|---|---|
| hOct4 | 5'-GGAGAATTTGTTCCTGCAGTGC |
| | 5'-AGAACCACACTCGGACCACATC |
| hSox2 | 5'-TCACGCAAAAACCGCGAT |
| | 5'-TATACAAGGTCCATTCCCCCG |
| hNanog | 5'- GCATCCGACTGTAAAGAATCTTCA |
| | 5'- CATCTCAGCAGAAGACATTTGCA |
| hSmad2 | 5'- GACACCAGTTTTGCCTCCAGTAT |
| | 5'- TCCAGAGGCGGAAGTTCTGT |
| HALP | 5'-ACGAGCTGAACAGGAACAACGT |
| | 5'-CACCAGCAAGAAGAAGCCTTTG |
| hPax7 | 5'-GCAAATTGCTGTCCTGCTCA |
| | 5'-TGAAAACTGGTCACATCTGCCT |
| hMyf5 | 5'-ACCGATTCACAGCCTCGAACT |
| | 5'-TGTGTATTAGGCCCTCCTGGAA |
| hMyoD | 5'-ACAGCGCGGTTTTTTCCAC |
| | 5'-AACCTAGCCCCTCAAGGTTCAG |
| hDesmin | 5'- GGAGAGGAGAGCCGGATCA |
| | 5'- GGGCTGGTTTCTCGGAAGTT |
| hDystrophin | 5'- CATCACATCACTCTTCCAAGTTTTG |
| | 5'- CCTTGGCAACATTTCCACTTC |
| hMyogenin | 5'-TGGCAGGAACAAGCCTTTTC |
| | 5'-ACAGGCAGGTAGTTTTCCCCA |
| hMEF2 | 5'-ATTCCACCAGGCAGCAAGAA |
| | 5'-GGAGTTGCTACGGAAACCACTG |
| hMLP | 5'-AAGGCTCTTGACAGCACGACAG |
| | 5'-TGTCCATACCCGATCCCTTTG |
| hMHC | 5'-CCAGCACCTGGGCAAGTC |
| | 5'-CCACAACACCAGCATAGTGAATC |
| h α-SM actin | 5'- CAAGTGATCACCATCGGAAATG |
| | 5'- GACTCCATCCCGATGAAGGA |
| h β-actin | 5'-ATCGTCCACCGCAAATGCT |
| | 5'-AAGCCATGCCAATCTCATCTTG |
| mPAX7-F | 5'-ACCAAGCTTTCAAGTCCGCA |
| | 5'-GCCTTACATTCTGGAGGATGGA |
| mMyf-F | 5'-CTCTGAAGGATGGACATGACGG |
| | 5'-ACTGGTCCCCAAACTCATCCTC |
| mMyoD-F | 5'-TTCCGGAGTGGCAGAAAGTTAA |
| | 5'-TCAAGTCTATGTCCCGGAGTGG |
| mMyogenin-F | 5'-TATCCGGTTCCAAAGCCTCTG |
| | 5'-GCGGCAGCTTTACAAACAACA |
| mMEF2 | 5'-AACCCCAATCTTCTGCCACTG |
| | 5'-ATCAGACCGCCTGTGTTACCTG |
| mMLP | 5'-GCTGAACAAGTTACTGAGCGGC |
| | 5'-ATTTTGCACCTCCACCCCA |
| mMHC | 5'-CCCCGCCCCACATCTT |
| | 5'-GATTGACTGATTCTCCCTGTCTGTT |
| m GAPDH | 5'-TGT GTCCGTCGTGGATCTGA |
| | 5'-CCTGCTTCACCACCTTCTTGA |

Analysis was performed using the ABI Prism Sequence Detection System 7500 (PE Biosystems) with SYBR Green PCR Master Mix (Applied Biosystems, Foster City, CA). Temperature cycling conditions were based on the inset conditions, and the relative quantification was performed by the CT method. The results were normalized to beta-actin levels.

For immunocytochemistry (ICC), the cultured cells were fixed with 4% paraformaldehyde for 5 minutes. The cells were washed with PBS three times and blocked in 5% BSA for 1 hour to prevent non-specific antibody binding. Then, the cells were cultured with primary antibodies at 4 °C overnight, washed with PBS three times, and incubated with secondary antibodies at room temperature for 1 hour. The antibodies used were Nestin, MyoD, α-SM actin, α-actinin (all from Santa Cruz Biotechnology, Santa Cruz, CA) and Desmin (BD Biosciences). The secondary antibodies bound to Alexa Fluro 594 immunofluorescence were applied for 30 minutes and washed with PBS. Samples were mounted in ProLong Gold antifade reagent (Invitrogen) with 4,6-diamino-2-phenylindole (DAPI) for staining. C2C12 cell line was used as a positive control and human fibroblasts were used as a negative control.

### Example 3: Incapacitation of Urethral Sphincter and Injection of hAFSCs

Mice were treated according to National Institutes of Health Animal Care Guidelines, which were approved by the Animal Ethics Committee of the Medical School of Kyungpook National University. All experiments were performed using 4-week-old female ICR mice (2025 mg). Before the surgical injury to the urethral sphincter, the abdominal leak point pressure (LPP) and closing pressure (CP) were measured. Immediately, anesthesia was induced by intramuscular injection of Zoletil (30 mg/kg, virbac animal health, France) and Rumpun (10mg/kg, Bayer, Korea). A lower midline abdominal incision was performed and the pudendal nerves on both sides were found. The bilateral pudendal nerves were transected with surgical scissors under a microscope.

hAFSCs (1x10⁶) were injected using a 26G Hamilton microsyringe (Hamilton Company, Reno, NV) with microscopic guidance. Three experimental groups were established: a control group (Ctrl) underwent sham-operation and cell injection without neurectomy; a group Cell (-) underwent pudendal neurectomy and injection of saline; and a group Cell(+) underwent pudendal neurectomy and injection of hAFSCs.

### Example 4: Preparation of Hydrogel for Injection of hAFSCs

An alginate solution (3%) was prepared by dissolving 300 mg of alginic acid sodium salt in 10 ml of PBS (pH 7.4) and completely mixed using a homogenizer. To prepare a Pluronic F-127 (PF-127) solution (25%), 5 g of polymer was dispersed and stirred in 20 ml of PBS. Partially dissolved PF-127 solution was stored in a refrigerator at 0 to 4 °C or using an ice bath until the entire polymer was completely dissolved. 20 µg of hyaluronic acid was dissolved in 1000 µl of distilled water. The mixing ratio of alginate/PF-127/hyaluronic acid was 6:6:1, and the mixture was homogeneously mixed for 5 minutes and left in a refrigerator for 6 hours. A sodium hyaluronate solution was prepared by dissolving 100 mg of sodium hyaluronate in 10 ml of PBS by the same process. Finally, Ca²⁺ (CaCl₂, 0.2%) was prepared by dissolving 100 mg of CaCl₂ in 50 ml of PBS. To prepare a cell-containing polymer solution, amniotic fluid stem cells at 1x10⁶ were slowly mixed and added to alginate/PF-127/hyaluronic acid.

### Example 5: Urodynamic Test

Leak point pressure (LPP) and closing pressure (CP) were measured at one, two and four weeks after injection using the vertical tilt/intravesical pressure clamp model. The spinal cords of anesthetized animals were transected at the T9 level. A catheter with a fire-flared tip (PE-90) was inserted into the bladder dome, and the abdominal wall was sutured. The mice were then placed on a tilt table in the vertical position, and saline was instilled into the bladder. The intravesical pressure was increased in 13 cm H₂O steps from 0 cm H₂O upward until visual identification of the leak point height. The averages of three consecutive LPP and CP measurements were taken.

### Example 6: Histological, Immunohistochemical, Molecular, and Immune response analysis

After the urodynamic test, the urethras were harvested for each time. Injected human cells were identified using anti-human nuclear antibody (HuNu, Chemicon) and the stem/myogenic lineage cells present in the urethral sphincter were analyzed. To determine an appropriate cell number for injection, 10⁴, 10⁵, and 10⁶ cells/sphincter were used. Optimal cell number was determined through the analysis of stem cells and myogenic antibodies (Nestin, Myod, and α-SMA). Through the histological analysis, *in vivo* tumor formation was also observed. The differentiation of injected hAFSCs into myocytes was determined through real-time PCR using human primers, and the host reaction required for cell therapy was measured using mouse myogenic primers. Protein expression was determined by IHC using Nestin, Myod, α-SM, and α-actininantibodies after two weeks and four weeks. To evaluate the immunogenicity of injected hAFSCs, the expression of HLA-DR on the cell surface was determined using FACS and T lymphocyte activation marker (CD8) IHC at one week after injection.

### Example 7: MNPs@SiO₂ Labeling of hAFSCs and in vivo Tracking through Optical Images

Magnetic nanoparticles were used to track the injected hAFSCs. Cobalt ferrite silica core-shell nanoparticles containing RITC [MNPs@SiO₂ (RITC)] were provided by Dr. Jae-sung Bae (Kyungpook National University, Daegu, Korea). For labeling, hAFSCs (10⁴) were cultured in 24 wells to reach 70% confluence, and MNPs@SiO₂ (RITC) nanoparticles were added for 24 hours. To establish effective uptake of nanoparticles, various concentrations were applied such as 0.01, 0.05, 0.1 or 0.2mg/mL. To evaluate time-dependent labeling efficiency, ICC staining was performed every 6 hours, and *in vivo* localization of MNPs@SiO₂ (RITC) was measured. To analyze the localization of nanoparticles labeled in hAFSCs *in vitro,* hAFSCs at 5x10⁴ cells were seeded in 35-mm tissue culture dishes containing growth media. When the cells reached about 70% confluence, MNPs@SiO₂ (RITC) (0.1mg/mL) was added, cultured for 3 hours, and suspended in FACS buffer (n=3). Labeled cells were passaged seven times to measure the maintenance ratio in the next passages. For optical imaging, cells were labeled with MNPs@SiO₂ (RITC) (0.2 mg/mL) at 37 °C for 3 hours. After anesthesia, 1x10⁶ nanoparticle-labeled cells were injected into urethral sphincter mice (n=3). After injection, optical images were obtained using Pro imaging system (Princeton Instrument, Trenton, NJ), and filters (Omega Optical, Brattleboro, VT) were set for RITC. Images were analyzed using Princeton Instrument software (winview/32 Metavue), and spectral unmixing algorithms were used to eliminate non-specific autofluorescence. The effect of the concentration (0.01∼0.2 mg/mL) of nanoparticles on the cell viability was measured using MTS assay.

### [Results]

### 1. Characterization of Human Amniotic Fluid Cells and Isolation of c-kit (+) cells

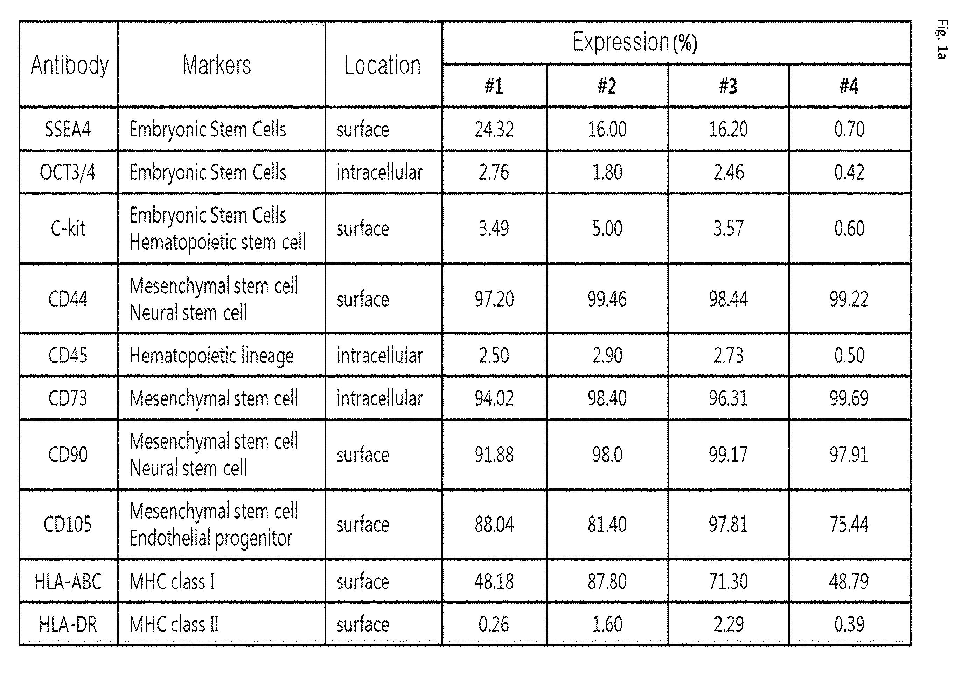

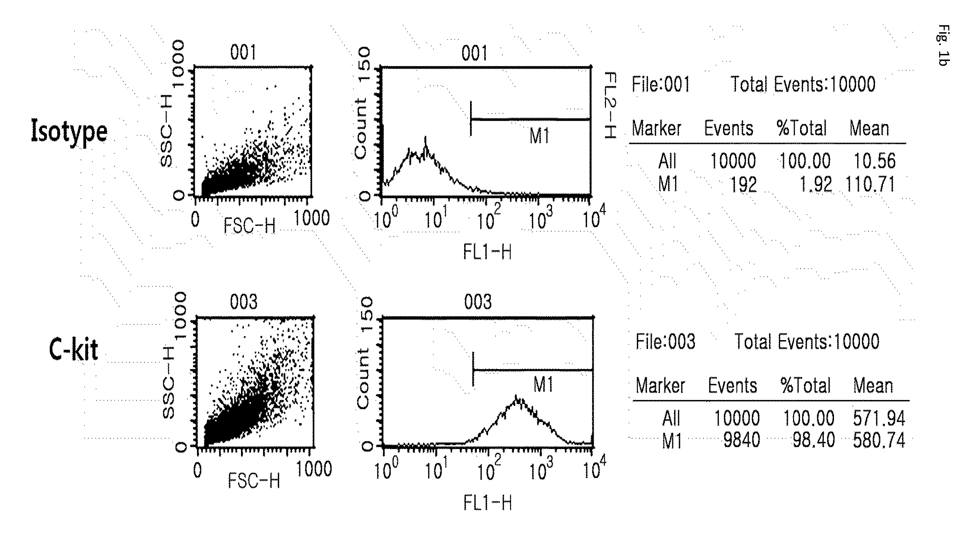

Cells were isolated from four different amniotic fluid samples and analyzed by FACS system (FIG. 1A). Human amniotic fluid cells expressed embryonic stem cell markers such as Oct-4, c-kit, and SSEA-4, from which it is interpreted that these cells have pluripotential capability. These cells were positive for mesenchymal stem cell markers, neural stem cell markers, and/or endothelial progenitor cell markers (CD44, CD73, CD90 and CD105), but negative for the hematopoietic lineage marker (CD45). In the case of immune response-related markers, cells were negative for Class II major histocompatibility antigen (HLA-DR). Class I c-kit (+) cell population was 0.6∼5.0% of the entire cell group. More than 90% of Class II c-kit (+) cells were positive for c-kit.

### 2. Myogenic Characteristics of hAFSCs in vitro

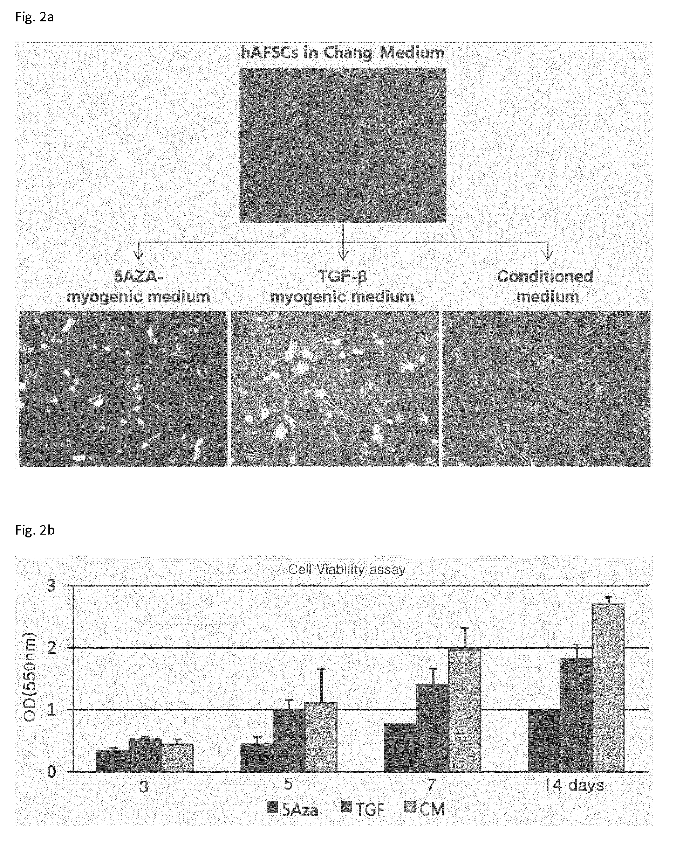

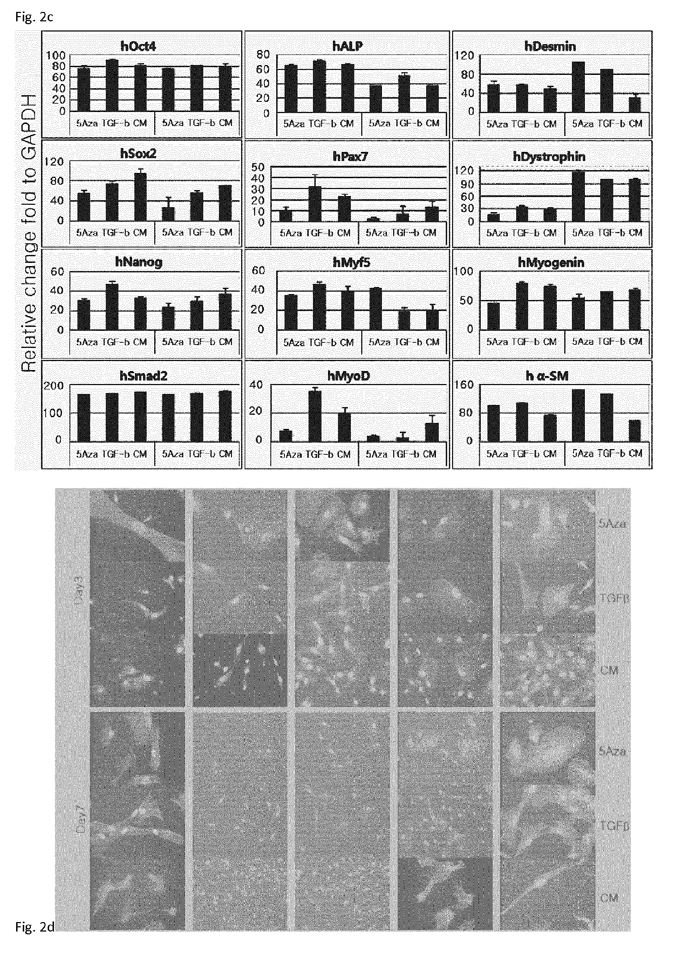

It was found that when hAFSCs at passage 5 were cultured in regular myogenic media) (5-azaC condition) for 7 days, cells expressed myogenic specific markers. The following two additional conditions were evaluate to determine the differentiation of hAFSCs into myocytes: TGF-β and conditioned medium. The cultured cells were morphologically similar such as elongated morphology (FIG. 2A-a, b, and c). In the cell viability assay through MTS assay, the hAFSCs cultured with 5-azaC and TGF-β showed significant cytotoxicity compared to CM (FIG. 2B). These results indicate that the culture of hAFSCs in CM showed mild myogenic conversion. In the real-time PCR analysis (FIG. 2C), stem cell and myogenic lineage markers in three different media showed various gene expression levels. At 3 days after culture, the expression of stem cell and early myogenic markers increased, and at 7 days, the expression of the mid to late myogenic markers was dominant. These results indicate that hAFSCs can differentiate into myogenic lineage cells in proposed media. Although the group treated with 5-azaC and TGF-β showed relatively high myogenic gene expression, it is considered that the CM medium is an appropriate myogenic condition for hAFSCs in terms of cell viability. Immunohistochemical (IHC) staining was performed to determine the results of gene expression (FIG. 2D).

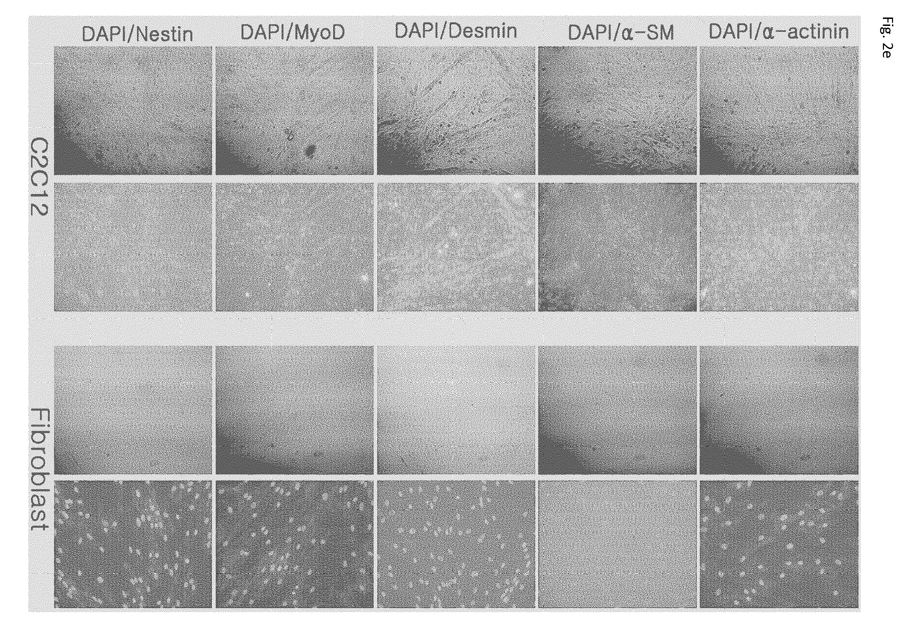

More than 90% of the cultured hAFSCs were stained positively for myogenic markers. Nestin was expressed with stem cell markers for the entire culture period. Cells cultured for 3 days expressed myogenic markers at the same time. At 7 days, the early markers such as MyoD and Desmin became weak, while the late markers such as α-SM actin and α-actininwere more expressed than before. Characteristically, the cells expressed significantly α-actinin-labeled thick filaments, and cells with spindle shapes were found in the group treated with CM. C2C12 cell line was used as a positive control and human fibroblasts were used as a negative control for differentiation into myoblasts (FIG. 2E).

### 3. Identification of Human Cells and Optimal Cell Number in vivo

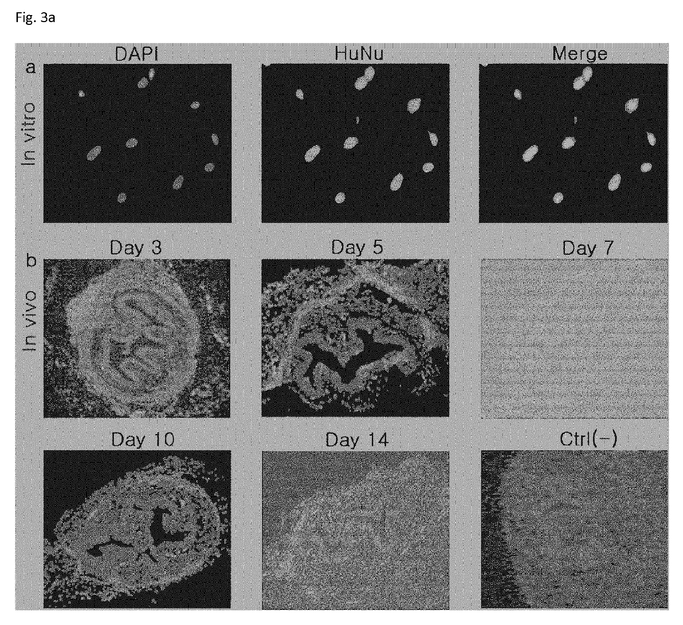

Human nuclear-specific antibody (HuNu) was used to determine the presence, migration, and duration of injected human cells. Human nuclei stained with anti-HuNu were matched with DAPI *in vitro* (FIG. 3A-a). The presence of human nuclei injected into the urethral sphincter was not found *in vivo* (FIG. 3A-b). Positive staining was locally observed at the injection site at 3 days, and the intensity decreased gradually and lasted up to 14 days. No HuNu-positive cells were found in cell walls of PBS-injected urethral sphincter.

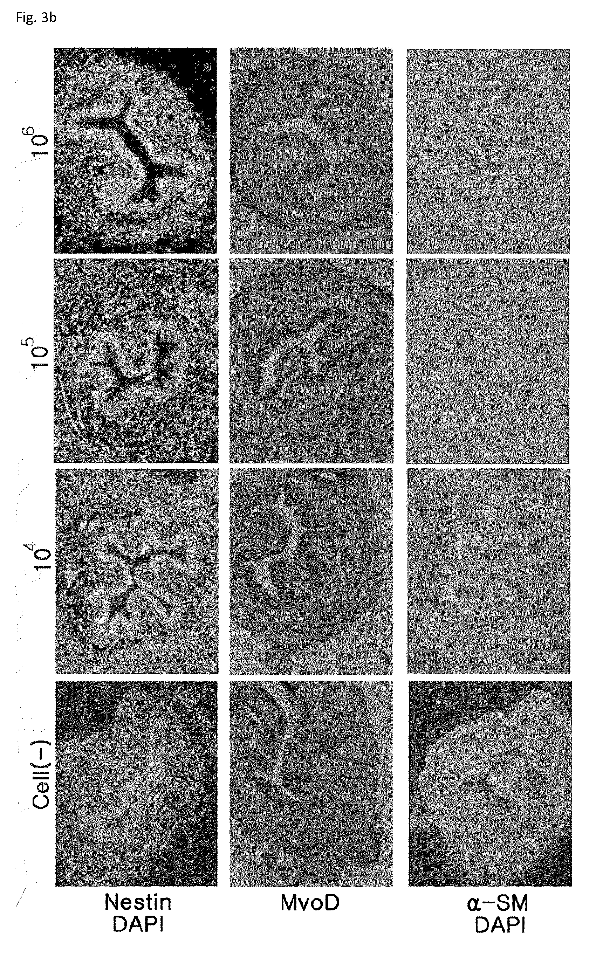

Among three treated groups with different cell numbers, the group with 10⁶ injected cells formed muscle bundles and showed effective urethral sphincter regeneration (FIG. 3B). The presence of a large amount of Nestin, MyoD and α-SM indicates the enhanced regeneration in the injured urethral sphincter according to the cell number. The group in which no cells were seeded and the group in which a small amount of cells were seeded showed insufficient regeneration without muscle tissues, limited smooth muscle bundle formation, and some irregular flaps on the outside of transitional epithelium. Due to technical limitations, the injection of 10⁷ cells was impossible. Thus, the optimal cell number in the mouse models was determined as 10⁶ cells.

### 4. Histological, Immunohistochemical, Molecular in vivo Analysis

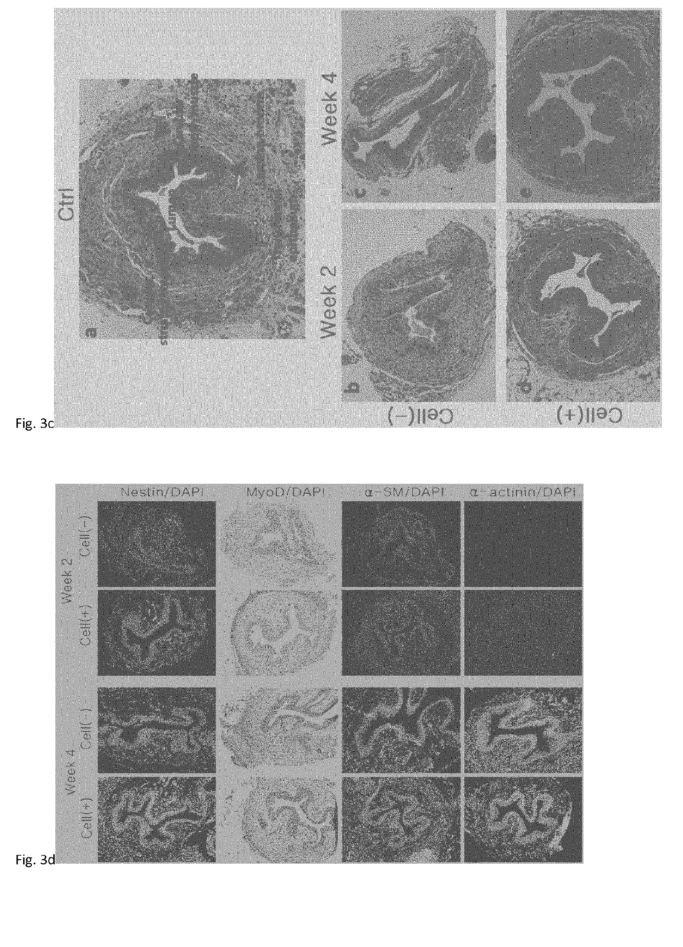

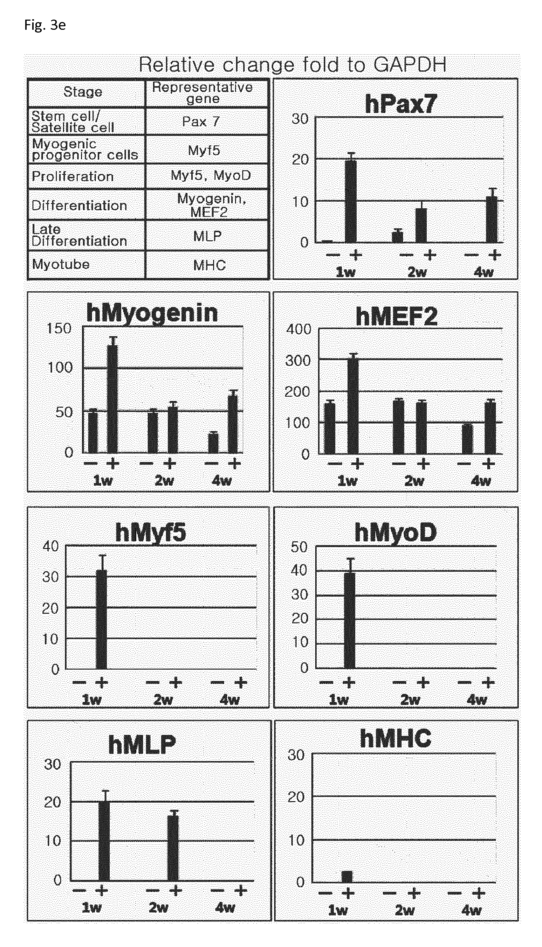

An additional study was performed using a reasonable cell number. Mouse urethral sphincter included smooth muscles and skeletal muscles (FIG. 3C-a). Urethral sphincter area injured by neurotomy was determined as an atrophic tissue, like contracted muscles, and had no smooth muscle and straight muscle laminar structure at 2 weeks and 4 weeks (FIG. 3C-b and c). The cell-injected group showed the regeneration of circular muscle mass and thick packed layers (FIG. 3C-d and e). The cell-transplanted organ maintained the pattern and effect of normal urethral sphincter shape. The increased urethral sphincter in all experimental groups was considered as evidence of complete muscle regeneration. These results are histological evidence of the cellular therapeutic effects on hAFSCs injected into urethral sphincter mouse models. No tumors were found in the cell (+) group, which indicates that the transplanted hAFSCs can adjust the cell growth in *in vivo* conditions and maintain normal phenotype.

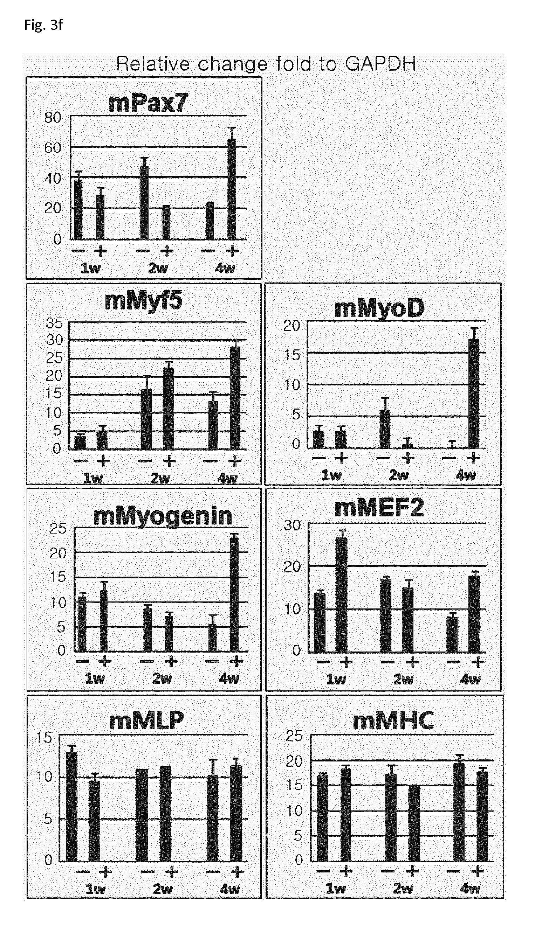

Stem cell and myogenic markers were determined using IHC. At 2 weeks, Nestin, MyoD, and α-SM increased in the cell-injected group, and α-actinin-positive cells formed the layered muscle structures showing the normal urethral sphincter structure (FIG. 3D). At 4 weeks, in the cell (+) group, the expression of the early markers (Nestin and MyoD) on the smooth and straight muscle layer decreased, and the late marker (α-SM and α-actinin)-positive cells were continuously maintained. On the contrary, in the cell (-) group, these proteins were weakly expressed. In the real-time PCR analysis, the injected human stem cells continuously expressed myogenic lineage-related genes (FIG. 3E). Gene expression using human primers relatively increased in the early stage, but decreased gradually over time. Although the injected cells had stemness properties, these cells differentiated into myogenic lineage cells in *in vivo* conditions. The expression of myogenic genes was analyzed using mouse primers, and the cell-treated group increased the expression of mouse genes compared to the cell (-) group (FIG. 3F). At 4 weeks, most of specific genes were highly expressed from the early to late markers. These results are considered that hAFSCs affect the host cell differentiation through unclear mechanism.

### 5. Immune Tolerance

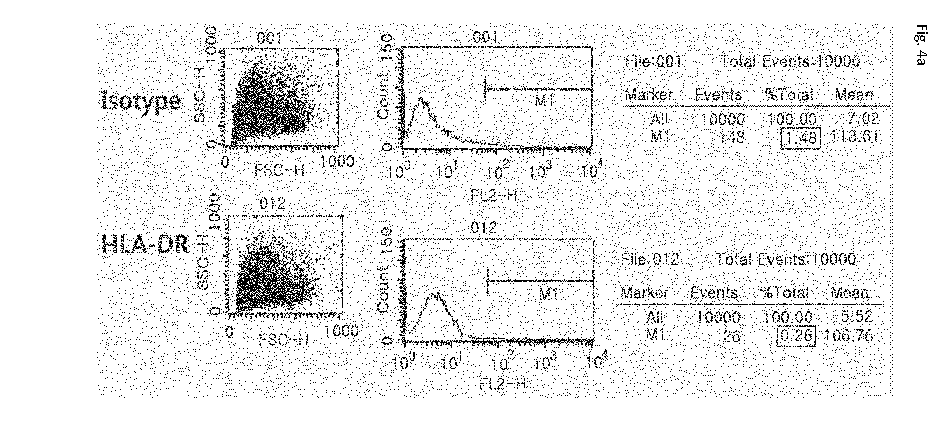

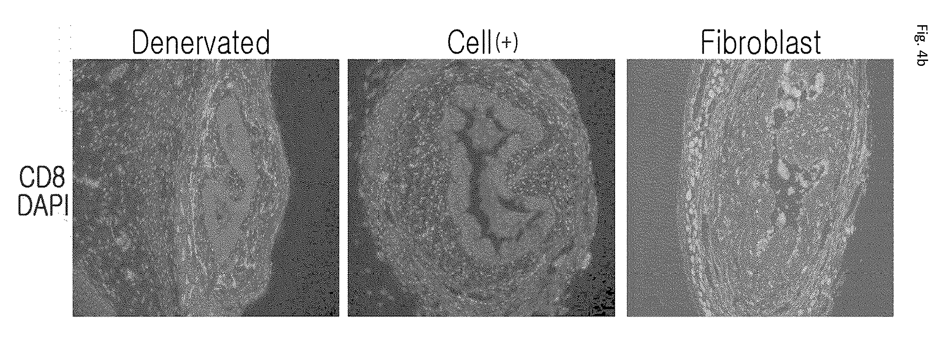

To analyze the functional immune, surface phenotypes of hAFSCs were analyzed using phycoerythrin-conjugated antibody against HLA-DR. hAFSCs weakly expressed HLA-DR (0.26%, 2.29%), and there was no significant difference in surface phenotype compared to samples, while there was a small change (FIG. 4A). The average ratio of positive cells was lower than that of the isotype Ab control. hAFSCs were injected into mouse urethral sphincters and active CD8 lymphocytes were stained at 1 week after injection (FIG. 4B). In the cell-injected group, CD8 lymphocytes (red) showed significantly weak reaction at the injection site, which indicates that hAFSCs did not induce immune reaction. On the contrary, the urethral sphincter which was denervated and into which human fibroblasts were injected showed significant CD8 lymphocyte reaction.

### 6. Optical imaging for in vivo tracking of injected cells

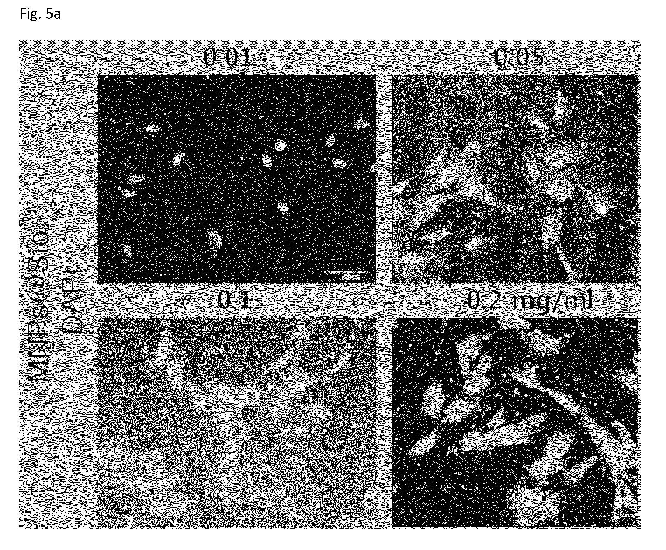

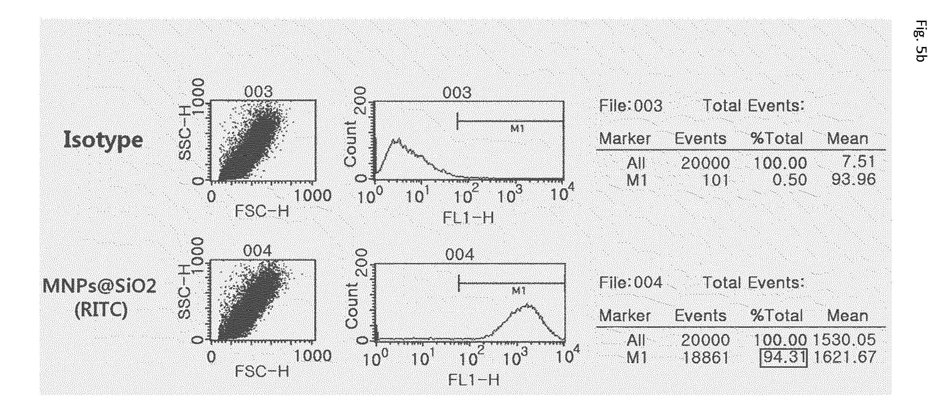

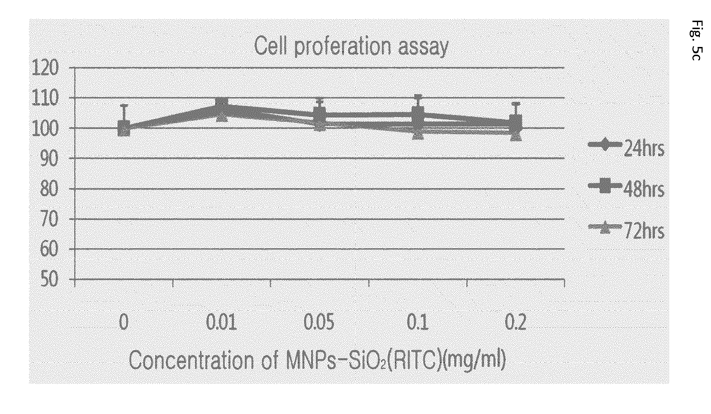

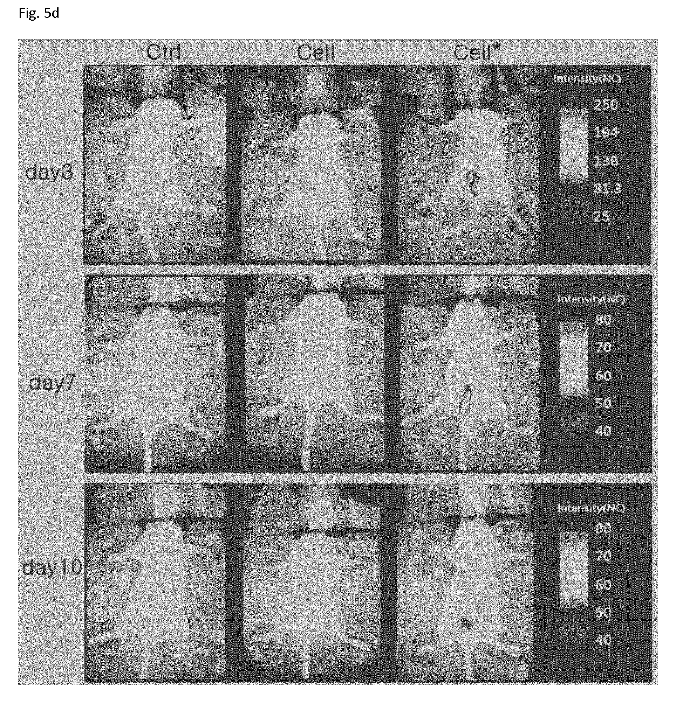

The optimum concentration of MNPs@SiO₂ (RITC) for hAFSCs, which provided sufficient images *in vitro,* was determined at 0.2 mg/ml using a fluorescence microscope (FIG. 5A). The signal intensity increased over time, and reached the highest value at 24 hours, and then there was no significant change in intensity up to 72 hours. With respect to the labeling efficiency at 24 hours, 94.31% of the cells in the entire group showed the RITC signal (FIG. 5B). Nanoparticles were localized in cytoplasm and clearly detected in a merged image. MTS cell proliferation assay showed that the viability of hAFSCs treated with 0.01, 0.05, 0.1 and 0.2 mg/mL MNPs@SiO₂ (RITC) for 24, 48 and 72 hours was similar over the entire period, which indicates that MNPs@SiO₂ (RITC) did not induce cytotoxicity in hAFSCs (FIG. 5C). Referring to the effects of nanoparticles on the cell cycle progression in hAFSCs, no nanoparticle-induced cell cycle arrest was found. MNPs@SiO₂ (RITC)-labeled cells were injected into the urethral sphincter (1x10⁶ cells). Optical images show that signals were expressed in the right side of the injection site of the labeled cells, while the control group and the non-labeled cells were not expressed. Over time, the signal intensity decreased slowly until 10 days (FIG. 5D).

### 7. Urodynamic Test

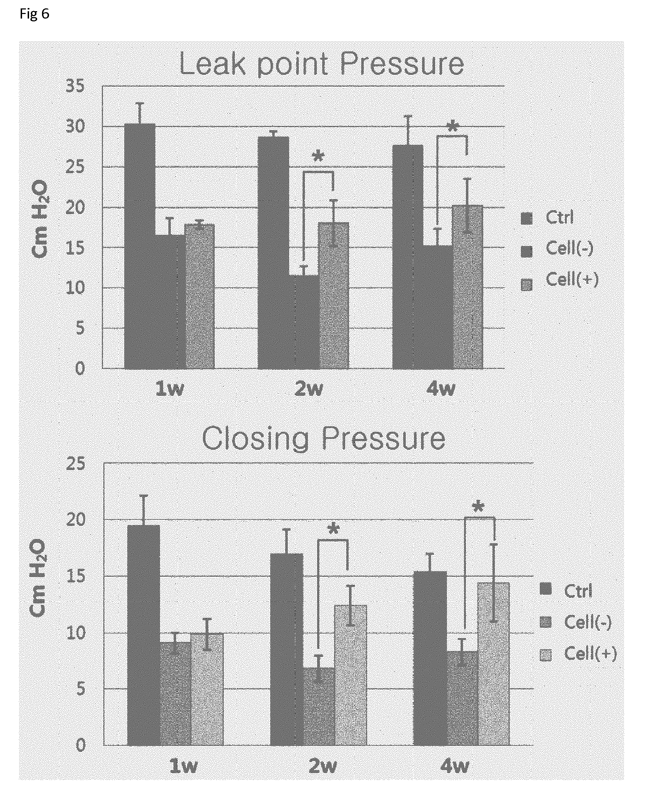

At one week, in the Ctrl, Cell (-) and Cell (+) groups, the LPP was 30.25±2.56 cmH₂O, 16.55±2.10 cmH₂O, and 17.90±0.49 cmH₂O, and the CP was 19.45±2.67, 9.13±0.87, and 9.88±1.34 cmH₂O. At two weeks, the LPP was 28.67±0.72, 11.59±1.18, and 18.06±2.78 cmH₂O, and the CP was 16.99±2.20, 6.85±1.09, and 12.42±1.71. At four weeks, the LPP was 27.59±3.64, 15.24±2.10, and 20.24±3.25 cmH₂O, and the CP was 15.38±1.64, 8.35±1.10, and 14.4±3.40. After one week of treatment, there was no difference in LPP and CP in the Cell (-) and Cell (+) groups. On the contrary, at two weeks and four weeks, the LPP and CP in the Cell (+) group were significantly higher than those of the Cell(-) group (P=0.05) (FIG. 6).

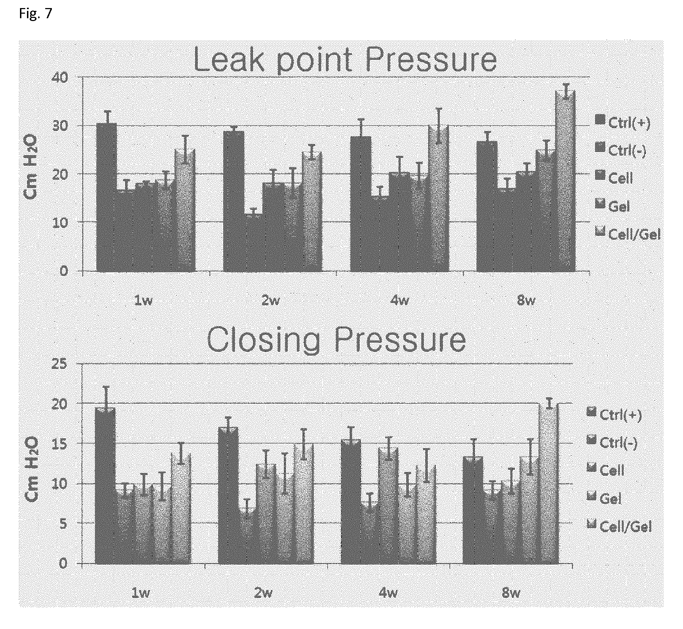

Moreover, the cells mixed with a hydrogel complex comprising alginate, pluronic acid, and hyaluronic acid showed significant restoration of LPP and CP compared to the cell group (FIG. 7)

The invention has been described in detail with reference to preferred embodiments thereof. However, it will be appreciated by those skilled in the art that changes may be made in these embodiments without departing from the principles and spirit of the invention, the scope of which is defined in the appended claims and their equivalents.

## Claims

1. A composition containing amniotic fluid-derived stem cells for treating sphincter deficiency.

2. The composition of claim 1, wherein the sphincter is urethral sphincter.

3. The composition of claim 2, wherein the composition is used to treat urinary incontinence.

4. The composition of claim 1, wherein the stem cells are contained in an amount of 10⁵ to 10⁶ cell/sphincter.

5. A composition comprising a hydrogel containing amniotic fluid-derived stem cells for treat sphincter deficiency.

6. The composition of claim 5, wherein the hydrogel is an alginate/PF-127/hyaluronic acid complex.

7. The composition of claim 6, wherein the ratio of the alginate, PF-127, and hyaluronic acid is 6:6:1.

8. The composition of claim 5, wherein the composition is formulated in the form of injections.

9. The composition of claim 5, wherein the composition is directly injected *in vivo* such that the stem cells differentiate in situ into myocytes *in vivo.*

10. A medium containing human skeletal muscle cell supernatant for inducing differentiation of stem cells into myocytes.

11. The medium of claim 10, wherein the stem cells are amniotic fluid-derived stem cell.
